(19)

**Europäisches Patentamt**
**European Patent Office**
**Office européen des brevets**

(11) **EP 3 982 372 A1**

(12) **EUROPEAN PATENT APPLICATION**

(43) Date of publication:
**13.04.2022  Bulletin 2022/15**

(51) International Patent Classification (IPC):
***G16H 20/60*** *(2018.01)*

(21) Application number: **20200890.0**

(52) Cooperative Patent Classification (CPC):
**G16H 20/60**

(22) Date of filing: **08.10.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ivanov, Bogdan**
**077191 Ilfov (RO)**

(72) Inventor: **IVANOV, Bogdan**
**077191 ILFOV (RO)**

(74) Representative: **Ex Materia**
**2, rue Hélène Boucher**
**78280 Guyancourt (FR)**

(54) **PROCESS OF SELECTING FOODSTUFFS TO MEET PERSONALIZED NUTRITIONAL NEEDS**

(57)     The invention related to a process of selecting foodstuffs (1000) for a diet of a subject that meets nutrients needs of the subject for a given duration (Dg). The process (1000) comprises a step of calculating (1100) a recommended intake interval (Rini) for each nutrient of a plurality of nutrients according to at least one characteristic of the subject, a step of determining (1200) an actual intake value (NAinv) for each nutrient, a step of comparing (1300) the actual intake value (NAinv) with respect to the recommended intake interval (Rini), if the actual intake value (NAinv) is outside the recommended intake interval (Rini), then proceed to a step of modifying (1400) quantity of foodstuffs in order to have the actual intake value (NAinv) within or below the recommended intake interval (Rini), if at least one nutrient has an adjusted actual intake value (AAinv) below its recommended intake interval (Rini), then proceed to a step of computing (1500) a quantity of nutrient supplement (Qns) to be added to the adjusted actual intake value (AAinv).

[fig 1]

EP 3 982 372 A1

**Description**

The present invention relates generally to the field of human nutrition, more specifically to methods for providing individuals with a diet program based on each individual's unique profile.

There has been a considerable change in the dietary habits of people living in industrial regions since the second half of the twentieth century. Traditional diets that were largely plant based have been replaced by diets that are high in fat or calories and have a substantial content of animal-based foods. There has also been a general decrease in physical activity in industrial countries as a result of a progressive shift toward a more sedentary lifestyle.

Diet and physical inactivity are known to be key risk factors associated with weight gain or the development of obesity. Obesity is associated with health risks, such as high blood pressure, coronary heart disease or diabetes. The number of people being clinically diagnosed as overweight or obese is increasing. Obesity is already responsible for 2-8% of health costs and 10-13% of deaths in different parts of Europe according to the World Health Organization. Children are not spared since 1 in 3 11-year-olds is overweight or obese in Europe.

Besides obesity, an estimated 33 million people are at risk of malnutrition despite the abundance of food in Europe. Malnutrition can result in compromised immune responses, which may lead to increased risk of infections, poor wound healing, delayed recovery from illness and longer hospitalization. Studies show that up to one third of patients in hospital and nursing homes are at risk of undernutrition, as are 10% of individuals over the age of 65 in the European Union. In addition, individuals undergoing rapid growth, such as infants and adolescents, but also pregnant women, have higher nutritional needs than others, and are therefore more susceptible to the effects of poor nutrition.

As a result, nutritionists, dietitians, doctors specialized in diabetes, nutrition and metabolic diseases and other specialists develop daily nutrition plans that should solve the nutritional needs of patients based on basic biochemical analysis and patients' lifestyle. This solution is difficult to implement, time consuming and expensive, with low efficiency. Furthermore, factors that contribute to health status and susceptibility to medical conditions vary between populations and between individuals within populations, so it is often impossible for an individual to derive useful advice appropriate to his or her particular circumstances from such general reports and research.

The present invention aims to overcome at least one of the aforementioned drawbacks and to further lead to other advantages by proposing a new type of process for selecting foodstuffs for a diet of a subject.

The present invention provides a process of selecting foodstuffs for a diet of a subject that meets nutrients needs of the subject for a given duration, said process comprising:

- a step of calculating a recommended intake interval for each nutrient of a plurality of nutrients for the given duration according to at least one characteristic of the subject,
- a step of determining an actual intake value for each nutrient of the plurality of nutrients derived from subject's foodstuffs eating habits over an usual duration which is equal to the given duration,
- a step of comparing the actual intake value with respect to the recommended intake interval for each nutrient of the plurality of nutrients,
- if the actual intake value of at least one nutrient of the plurality of nutrients is outside its corresponding recommended intake interval, then proceed to a step of modifying quantity of foodstuffs for the diet in order to adjust the actual intake value of each nutrient of the plurality of nutrients within or below the corresponding recommended intake interval,
- if at least one nutrient of the plurality of nutrients has an adjusted actual intake value below its corresponding recommended intake interval after the step of selecting, then proceed to a step of computing a quantity of nutrient supplement of said one nutrient to be added to the adjusted actual intake value so that the quantity of nutrient supplement of said one nutrient added to the adjusted actual intake value of said one nutrient falls into the corresponding recommended intake interval.

The proposed process comprises at least five steps which ultimately allows one subject to obtain a diet over a given duration that meets one subject's nutrient necessary needs. Indeed, the process considers at least one characteristic of the person and furthermore the process uses nutrient supplements to meet very precisely one subject's nutrient necessary needs.

According to one embodiment, the at least one characteristic of the subject is a nutrigenetic profile of the subject and/or a physical trait of the subject chosen in the group comprising a mass, an age, a gender, a pregnancy state and/or a lactation state.

A nutrigenetic profile is a DNA test that identifies and characterizes gene variants associated with differential responses to nutrients. The nutrigenetic profile then relates this variation to disease states. In other words, the nutrigenetic profile linked gene expression related to the metabolism of a nutrient to an amount of said nutrient to be ingested on a daily basis for example.

According to one embodiment, the step of calculating the recommended intake interval of each nutrient of the plurality of nutrients for the given duration comprises a first substep of obtaining the recommended daily intake value for each nutrient and a second substep of creating the recommended intake interval of each nutrient of the plurality of nutrients for the given duration from the recommended daily intake value of each nutrient.

According to one embodiment, the recommended intake interval for each nutrient has a lower bound which is equal to the recommended daily intake value of the nutrient times the given duration.

According to one embodiment, the recommended intake interval for each nutrient has a upper bound which is a maximum chronic daily intake for the nutrient times the given duration.

According to one embodiment, the maximum chronic daily intake is provided by a food safety organization by using one or more physical traits. The food safety organization is for example the European Food Safety Agency or the U.S. Department of Health and Human Services or the UK Department of Health.

According to one embodiment, the recommended daily intake value is provided by the nutrigenetic profile or a food safety organization by using one or more physical traits. The food safety organization is for example the European Food Safety Agency or the U.S. Department of Health and Human Services or the UK Department of Health. According to one embodiment, the recommended daily intake value from the nutrigenetic profile takes precedence over the recommended daily intake value from the food safety organization. In other words, if the nutrigenetic profile is not available, one can use instead the recommended daily intake values provided by the food safety organization.

According to one embodiment, the step of determining the actual intake value for each nutrient of the plurality of nutrients derived from subject's foodstuffs eating habits comprises a first substep of evaluating quantities of actual foodstuffs eaten by the subject over the usual duration, and a second substep of converting the quantities of actual foodstuffs eaten by the subject into actual intake value for each nutrient of the plurality of nutrients.

According to one embodiment, the process comprises a step of assessing foodstuff preferences of the subject. Thus, it is easier for the subject to abide by the diet it includes subject's foodstuff preferences. In other words, the acceptance of the diet by the subject is higher than for any other diet because it takes into consideration the subject's foodstuffs preferences.

According to one embodiment, the step of assessing foodstuff preferences of the subject is conducted at the same time as the step of determining the actual intake value for each nutrient.

According to one embodiment, the process comprises a step of representing, in a single graph, the recommended intake interval of each nutrient and the adjusted actual intake value for each nutrient. This can facilitate the achievement of the step of modifying quantity of foodstuff for the diet. In addition, the actual intake value of each nutrient can be plotted on that graph along with adjusted actual intake values to even more facilitate the achievement of the step of modifying quantity of foodstuffs for the diet.

According to one embodiment, the given duration is a day, a week, a month, a quarter, a semester or a year. In other words, the given duration can be any time interval suitable to the subject. This allows a greater flexibility in the use of process according to the invention.

According to one embodiment, the process comprises a step of purchasing selected quantity of foodstuffs for the diet and/or modified quantity of nutrient supplement for the given duration. Then the subject can have all the right quantities of foodstuffs at once either automatically or manually.

According to one embodiment, the process comprises a step of proposing menus and/or recipes from the modified quantity of foodstuffs for the diet and calculated quantity of nutrient supplement within the given duration.

According to one embodiment, the process comprises a step of monitoring the evolution of health of the subject during the given duration.

According to one embodiment, the step of monitoring the evolution of the subject's health is made by basic biochemical analysis such as blood sample analysis and/or urine sample analysis.

It should be understood here, as well as in all that follows, by « basic biochemical analysis » that it is an analysis of a tissue sample or a fluid sample (for example urine, feces, blood or saliva) where one looks for sodium, potassium, chloride, bicarbonate, blood urea nitrogen (BUN), magnesium, creatinine, glucose, calcium and/or cholesterol and/or levels of immune cells. Regarding cholesterol levels, one can determine LDL and HDL cholesterol levels, as well as triglyceride levels.

According to one embodiment, the process can be a computer-implemented process.

The present invention also provides a computer program comprising instructions which, when the program is executed by a computer, cause the computer to carry out the process according to the present invention. It should be understood here, as well as in all that follows, by "computer" that it is an electronic system comprising at least one central processing unit. So, a computer can be for an example a personal computer or a mobile phone.

The present invention further provides a computer-readable medium comprising instructions which, when executed by a computer, cause the computer to carry out the process according to the present invention.

The present invention additionally provides a data carrier signal carrying the computer program of the present invention.

Other characteristics and advantages of the invention will become apparent from the following description and from several exemplary embodiments given by way of nonlimiting indication with reference to the appended schematic drawings, in which:

[fig 1] Figure 1 is flowchart of the process of selecting foodstuffs to meet personalized nutritional needs according to one embodiment of the invention;

[Fig 2] Figure 2 is an example of a plot used in the process for selecting foodstuffs according to the invention.

It should first be noted that if the figures show the invention in detail for its implementation, they can be used to better define the invention if necessary. It should also be noted that, in all the figures, similar elements and/or elements fulfilling the same function are indicated by the same number.

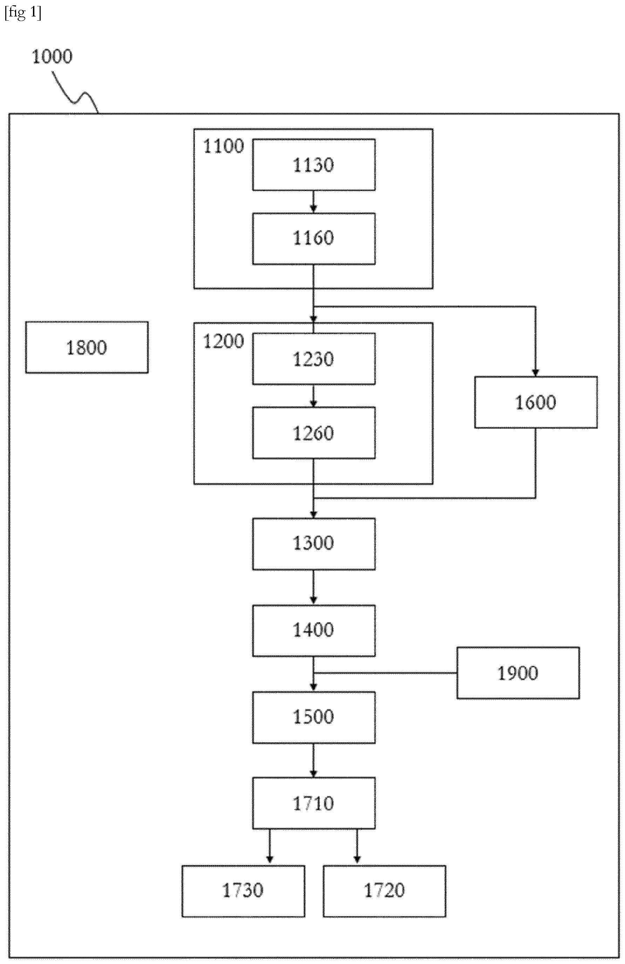

The process of selecting foodstuffs 1000 for a diet that meets nutrients needs of a subject for a given duration Dg is going to be described in relation with figure 1. The subject is a female, 26 years old, with psoriasis. In the following example, the given Dg duration is set to be 7 days. However, the given duration Dg can be a day, a week, a month, a quarter, a semester a year or any time interval suitable to the subject.

The process of selecting foodstuffs 1000 comprises a step of calculating 1100 a recommended intake interval Rini for each nutrient of a plurality of nutrients for the given duration Dg according to at least one characteristic CHA of the subject.

The plurality of nutrients comprises water, protein, carbohydrates, fats, minerals which are calcium, chloride, copper ,fluoride, iodine, iron, magnesium, manganese, molybdenum, phosphorus, potassium, selenium, sodium, zinc, and vitamins which are biotin, choline, cobalamin (vitamin B12), folate, niacin, pantothenic acid, riboflavin, thiamin, vitamin A, vitamin B6, vitamin C, vitamin D, vitamin E, vitamin K.

In a first embodiment, the at least one characteristic CHA is the nutrigenetic profile of the subject i.e. the 26 years old female with psoriasis.

The step of calculating 1100 a recommended intake interval Rini of each nutrient of the plurality of nutrients for the given duration Dg starts with a first substep of obtaining 1130 the recommended daily intake value RDinv for each nutrient. The recommended daily intake value RDinv for each nutrient is provided by the nutrigenetic profile and is found in table 1 thereafter. The recommended daily intake values RDinv are in grammes per day (g/day), in milligrammes per day (mg/day) or in microgrammes/day ($\mu$g/day), but they can be expressed in any other appropriate units.

The step of calculating 1100 then comprises a second substep of creating 1160 the recommended intake interval Rini of each nutrient of the plurality of nutrients for the given duration Dg from the recommended daily intake value RDinv of each nutrient. The second substep of creating 1160 follows the first substep of creating 1130.

The second substep of creating 1160 is to compute a the recommended intake interval Rini of each nutrient has a lower bound Rini,min of the recommended intake interval Rini and, if necessary, a upper bound Rini,max of the recommended intake interval Rini.

The lower bound Rini,min corresponds to the recommended daily intake value RDinv of a nutrient times the given duration Dg:

$$\mathrm{Rini,min = RDinv \times Dg}$$

In this first embodiment, the upper bound Rini,max is proportional to a maximum chronic daily intake MCD of a nutrient (from all sources) judged to be unlikely to pose a risk of adverse health effects to humans . It is usually a mass per day. So, the upper bound Rini,max corresponds to the maximum chronic daily intake MCD of the nutrient times the given duration Dg.

The maximum chronic daily intake MCD for a nutrient can be provided by a food safety organization by using one or more physical traits. The food safety organization is for example the European Food Safety Agency or the U.S. Department of Agriculture or the UK Department of Health. Here the maximum chronic daily intake MCD of each nutrient has been retrieved from European Food Safety Agency which has named it tolerable upper intake level UL. For that purpose, two characteristics CHA each being a physical trait were used. One is the gender of the subject and another one is the age of the subject.

Thus, the upper bound Rini,max is calculated as follows:

$$\mathrm{Rini,max = MCD \times Dg}$$

If the maximum chronic daily intake MCD is not available mainly because the scientists didn't discover until now negative effect of those nutrients intake then the there is no Rini,max. In other words, Rini,max is set to infinity and one can eat as much as one wants.

If one takes vitamin A as in table 1, RDinv is 700µg/day according to the genetic profile. Consequently Rini,min is (700 x 7) = 4900µg for the given duration Dg. The MCD given by the European Food Safety Agency is 3000µg/day, so the Rini,max is (3000 x 7) = 21000µg for the given duration Dg. So Rini for Vitamin A over a period of 7 days contains all numbers lying between 4900µg and 21000µg, 4900µg and 21000µg included.

If one takes potassium as in table 1, RDinv is 3500mg/day according to the genetic profile. Consequently Rini,min for the given duration is (0,5 x 7) = 24500mg. The MCD of potassium is not available from European Food Safety Agency. So Rini for potassium will contain all values above 24500mg, 24500mg being included which can be written [24500mg ; +∞[.

These calculations can be performed for all nutrients. All Rini have been summarized in table 1. Whenever the MCD for a nutrient is not available, the cell states 'not available' and no value has been assigned to the corresponding Rini,max.

Table 1:

| Nutrients | Rinv | MCD | Rini, min | - | Rini, max | Quantity derived from food stuff quantity before step 1200 /day | NAinv | Quantity derived from foodstuff quantity after step 1200 / day | AAinv |
|---|---|---|---|---|---|---|---|---|---|
| Carbohydrates (g) | 196,4 | 262,0 | 1374,8 | - | 1834 | 152,9 | 1070,3 | 180,0 | 1260 |
| Protein (g) | 66,0 | Not available | 462 | - | | 897 | 627,9 | 85,7 | 599,9 |
| Fat (g) | 38,0 | 67.8 | 266 | - | 474,6 | 83,6 | 585,2 | 76,5 | 535,5 |
| Fiber (g) | 25.0 | Not available | 175 | - | | 19,2 | 134,4 | 235 | 164,5 |
| Omega 3 a-linolenic acid(g) | 1,1 | Not available | 7,7 | - | | 2,5 | 17,5 | 2,9 | 20,3 |
| Omega 6 Linoleic acid (g) | 7,7 | 12 | 53,9 | - | 84 | 8,8 | 61,6 | 7.9 | 55,3 |
| Vit,A Retinol (µg) | 700,0 | 3000 | 4900 | - | 21000 | 243,9 | 1707,3 | 195,7 | 1369,9 |
| Vit,B1 Thiamin (mg) | 0,5 | Not available | 3,5 | - | | 1,9 | 13,3 | 1,9 | 13,3 |
| Vit,B2 Riboflavin (mg) | 2,2 | Not available | 15,4 | - | | 1,5 | 10,5 | 1,3 | 9,1 |
| Vit,B3 Niacin (mg) | 14.0 | 35 | 98 | - | 245 | 21,1 | 147,7 | 18,4 | 128,8 |
| Vít,B5 Pantothenic acid (mg) | 5,0 | Not available | 35 | - | | 5,9 | 41,3 | 5,1 | 35,7 |
| Vit,B6 Pyridoxine (mg) | 1,3 | 25 | 9,1 | - | 175 | 2 | 14 | 2,1 | 14,7 |
| Vit,B7 Biotin (µg) | 40,0 | Not available | 280 | - | | 38,2 | 267,4 | 46,3 | 324,1 |
| Vit,B9 Folate (µg) | 400,0 | 1000 | 2800 | - | 7000 | 295,1 | 2065,7 | 487,7 | 3413,9 |

(continued)

| Nutrients | Rinv | MCD | Rini, min | - | Rini, max | Quantity derived from food stuff quantity before step 1200 /day | NAinv | Quantity derived from foodstuff quantity after step 1200 / day | AAinv |
|---|---|---|---|---|---|---|---|---|---|
| Vit,B12 Cobalamin (µg) | 4,8 | Not available | 33,6 | - | | 5,8 | 40,6 | 4,4 | 30,8 |
| Vit,C Ascorbic acid (mg) | 80,0 | 2000 | 560 | - | 14000 | 108,5 | 759,5 | 165,9 | 1161,3 |
| Vitamin D (µg) | 15,0 | 100 | 105 | - | 700 | 4,3 | 30,1 | 8,9 | 62,3 |
| Vitamin E (mg) | 15,0 | 300 | 105 | - | 2100 | 10,4 | 72,8 | 13,7 | 95,9 |
| Vitamin K (µg) | 90,0 | Not available | 630 | - | | 75 | 525 | 139,7 | 977,9 |
| Copper (mg) | 1,3 | 5 | 9,1 | | 35 | 1,4 | 9,8 | 1,9 | 13,3 |
| Iron (mg) | 18,0 | 45 | 126 | - | 315 | 12,4 | 86,8 | 15,8 | 110,6 |
| Iodine (µg) | 150,0 | 600 | 1050 | - | 4200 | 98,4 | 688,8 | 65,2 | 456,4 |
| Manganese (mg) | 3,0 | 11 | 21 | - | 77 | 4,9 | 34,3 | 6,2 | 43,4 |
| Selenium (µg) | 85,0 | 300 | 595 | - | 2100 | 76,9 | 538,3 | 94,1 | 658,7 |
| Zinc (mg) | 8,0 | 40 | 56 | - | 280 | 11,8 | 82,6 | 9,4 | 65,8 |
| Calcium (mg) | 1250,0 | 2500 | 8750 | - | 17500 | 682,9 | 4780,3 | 664,9 | 4654,3 |
| Chloride (g) | 2,3 | Not available | 16,1 | - | 21,7 | 2,7 | 18,9 | 2,6 | 18,2 |
| Phosphorus (mg) | 550,0 | 4000 | 3850 | - | 28000 | 1409,4 | 9865,8 | 1471,4 | 10299,8 |
| Magnesium (mg) | 380,0 | Not available | 2660 | - | | 360,8 | 2525,6 | 472,9 | 3310,3 |
| Potassium (mg) | 3500,0 | Not available | 24500 | - | | 3323,6 | 23265,2 | 4163,4 | 29143,8 |
| Sodium (g) | 1,5 | 2 | 10,5 | - | 14 | 1,7 | 11,9 | 1,8 | 12,6 |

In an alternative embodiment, some of the maximum chronic daily intake MCD are a multiple of the recommended daily intake value RDinv, e.g. 2Rini or 4Rini.

A step of determining 1200 an actual intake value NAinv for each nutrient of the plurality of nutrients derived from subject's foodstuffs eating habits over a usual duration Tu which is equal to the given duration Dg is then performed. So in the present example Tu is equal to 7 days, i.e 1 week. For example, it can be derived from what the subject has eaten the week before starting the process of selecting foodstuffs 1000. In another example where Dg is a month, Tu would be a month too and the actual intake value NAinv for each nutrient could be derived from what the subject has eaten the month before starting the process of selecting foodstuffs 1000.

The step of determining 1200 comprises a first substep of evaluating 1230 quantities of actual foodstuffs Qaf eaten by the subject over the usual duration Tu. In other words, the first substep of evaluating 1230 consists in collecting eating habits of the subject over the usual duration Tu. So one gets a picture of what foodstuffs the subject usually eats during the usual duration Tu.

The step of determining 1200 comprises a second substep of converting 1260 the quantities of actual foodstuffs Qaf eaten by the subject into actual intake value NAinv for each nutrient of the plurality of nutrients as it can be seen in table 2 thereafter. Indeed, the foodstuffs are made of nutrients. The conversion is made by using data from published literature. Such data can be summarized and made available by food safety organization such the U.S. Department of Health and Human Services or the UK Department of Health. The second substep of converting 1260 follows the first substep of evaluating 1230.

Table 2

| Foodstuff | Qaf (g/week) | Qmod (g/week) |
|---|---|---|
| Cheese hard | 40 | 0 |
| Cheese processed plain | 30 | 0 |
| Cheese white | 40 | 0 |
| Milk whole pasteurised | 450 | 0 |
| Yogurt whole milk plain | 70 | 0 |
| Bacon rashers back grilled crispy | 50 | 0 |
| Beef steak grilled | 250 | 0 |
| Chicken grilled | 120 | 0 |
| Ham | 60 | 0 |
| Lamb loin chops grilled | 60 | 0 |
| Pork, grilled, | 250 | 0 |
| Pork, roasted | 60 | 0 |
| Salmon smoked | 100 | 240 |
| Salt water fish steamed | 37.5 | 360 |
| Tuna canned | 37.5 | 75 |
| Sunflower seeds toasted | 25 | 5 |
| Courgette boiled | 150 | 360 |
| Gherkins | 50 | 100 |
| Potatoes old boiled | 150 | 240 |
| Almonds flaked and ground | 0 | 10 |
| Cashew nuts kernel only plain | 0 | 40 |
| Pine nuts, kernel only | 0 | 5 |
| Brussels sprouts | 0 | 100 |
| Cabbage white | 0 | 200 |
| Lettuce, arugula, raw | 0 | 175 |
| Tofu soya bean steamed | 0 | 100 |
| Houmous | 0 | 150 |
| Quinoa | 0 | 40 |
| Sweet potato, baked | 0 | 240 |
| Beans green boiled | 0 | 300 |
| Beans soya boiled | 0 | 300 |
| Beetroot | 0 | 150 |
| Cauliflower | 0 | 0 |

A step of comparing 1300 the actual intake value NAinv with respect to the recommended intake interval Rini

for each nutrient of the plurality of nutrients takes place after the step of determining 1200. If the actual intake value NAinv for each nutrient falls within the recommended intake interval then there is no need to change or adjust the diet of the subject.

In one embodiment when one collects eating habit over a period that is more than a week then the comparison is made on weekly basis. For example, if a foodstuff is consumed once every 2 weeks then one divides the quantity of that foodstuff by 2. In another example, when a foodstuff is consumed once every 4 weeks, we divided the quantity of that foodstuff by 4 to have all quantities on weekly basis.

If the actual intake value NAinv of at least one nutrient of the plurality of nutrients is outside its corresponding recommended intake interval Rini, then the process of selecting foodstuffs 1000 proceeds to a step of modifying 1400 quantity of foodstuffs for the diet in order to adjust the actual intake value NAinv of each nutrient of the plurality of nutrients within or below the corresponding recommended intake interval Rini. In other words, the quantities of actual foodstuffs Qaf serves has a starting point to adjust the actual intake value NAinv of each nutrient. One can then vary those quantities of actual foodstuffs Qaf up and down in order to reach quantities of nutrients, i.e. the adjusted actual intake value AAinv, that fall in or are below the recommended intake interval Rini.

If at least one nutrient of the plurality of nutrients has an adjusted actual intake value AAinv below its corresponding recommended intake interval Rini after the step of modifying 1400, then proceed to a step of computing 1500 a quantity of nutrient supplement Qns of said one nutrient to be added to the adjusted actual intake value AAinv so that the quantity of nutrient supplement Qns of said one nutrient added to the adjusted actual intake value AAinv of said one nutrient falls into the corresponding recommended intake interval Rini. This can be observed for vitamin A and vitamin D. Indeed, both adjusted actual intake values AAinv are below its corresponding Rini,min, so the subject will have to supplement its diet with vitamin A supplement and vitamin D supplement so as to get the right amount of vitamin A and vitamin D.

The process of selecting foodstuffs 1000 comprise a step of assessing 1600 foodstuff preferences of the subject. This step of assessing 1600 foodstuff preferences of the subject occurs at the same time as the step of determining 1200 the actual intake value NAinv for each nutrient. However, the step of assessing may be conducted at any other convenient step.

As it can be seen in table 2, before using the process of selecting foodstuffs 1000, the subject was not eating some foodstuffs like tofu soya bean steamed, and after the subject was. Tofu soya bean steamed was included in the diet because it was detected during the step of assessing 1600 that the subject liked Tofu soya bean steamed. The same goes for baked sweet potatoes. Conversely, cauliflower was not included because the subject indicated that it did not like it during the step of assessing 1600.

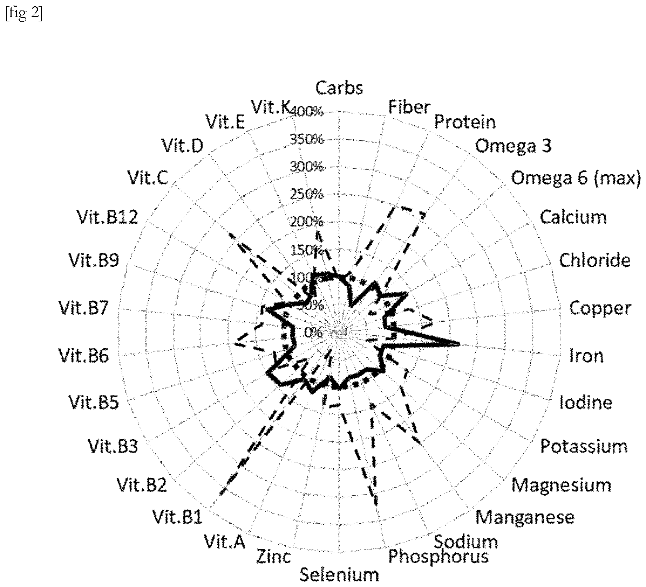

The process of selecting foodstuffs 1000 further comprises a step of representing 1900, in a single graph, the recommended daily intake value RDinv of each nutrient, the adjusted actual intake value AAinv for each nutrient as illustrated in figure 2. In figure 2, carbs means carbohydrates.

The graph is for example a radar chart where each radius is dedicated to a nutrient. Each radius is a percentage calculated versus the European Food Safety Authority recommended values which is considered to be 100%. The squared dashed line represents the recommended daily intake value RDinv of each nutrient with respect to the recommended daily intake value RDinv of that nutrient so it is 100% for all nutrients. The rectangular dashed line represents the actual intake value divided by the given duration Dg with respect to the recommended daily intake value RDinv of that nutrient. The plain line represents the adjusted actual intake value AAinv divided by the given duration Dg for each nutrient with respect to the recommended daily intake value RDinv for that nutrient.

If one looks at vitamin B7, the recommended daily intake value RDinv is $40\mu g$ which is considered 100% which is the plain line. The quantity of vitamin B1 derived from foodstuff before step 1200 per day is $38{,}9\mu g$. Thus the ratio of $38{,}9\mu g$ by $40\mu g$ gives 97,3%, as seen on the squared dotted line on the radar chart displayed by figure 2. And the quantity of vitamin B7 derived from foodstuff after step 1200 per day is $46{,}3\mu g$. Thus the ratio of $46{,}3\mu g$ by $40\mu g$ gives 116% which is indicated by the rectangular dotted line on the radar chart displayed by figure 2. In a non-illustrated embodiment, the radar chart comprises in addition the actual intake value NAinv for each nutrient divided by the given duration with respect to the recommended daily intake value Rinv for that nutrient. In a non-illustrated embodiment, the maximum chronic daily intake MCD for each nutrient is represented on the radar chart with respect to the recommended daily intake value Rinv for that nutrient.

The process of selecting foodstuffs 1000 comprises in addition a step of transforming 1710 the computed quantity of nutrient supplement Qns into commercialized quantities used by the manufacturers of nutrient supplement product to identify the exact quantity to be purchased.

The process of selecting foodstuffs 1000 also comprises a step of purchasing 1720 modified quantity of foodstuffs Qmod for the diet and/or calculated quantity of nutrient supplement Qns for the given duration Dg.

The process of selecting foodstuffs 1000 then comprises a step of proposing 1730 menus and/or recipes from the modified quantity of foodstuffs Qmod for the diet and from the calculated quantity of nutrient supplement Qns within the given duration Dg. As a consequence, the subject has a better proclivity to follow the diet over the given duration Dg.

The process of selecting foodstuffs 1000 can comprise a step of monitoring 1800 the evolution of health of the subject during the given duration Dg. The subject's health monitoring can be made by basic biochemical analysis such as blood sample analysis and/or urine sample analysis. Basically, the subject can undergo at least one basic biochemical analysis during the diet. In a preferred embodiment, the subject can undergo at least one basic biochemical analysis on a regular basis during the diet. Thus, results can be compared with each other to allow an excellent monitoring of the evolution of health of the subject during the given duration Dg.

In a second embodiment, the process of selecting foods 1000 is the same except that the step of calculating 1100 is only based on two characteristics CHA each being a physical trait. One is the gender of the subject and another one is the age of the subject.

Those physical traits were used to retrieve the recommended daily intake value RDinv from the European Food Safety Authority. The recommended daily intake value NAinv from the European Food Safety Authority is called dietary reference value. The European Food Safety Authority provides different flavors of dietary reference value comes in different flavor: population recommended intake PRI value which meets the needs of almost all population, average recommended AR value which meets the needs of half of the population, adequate intake AI value which is based on observation or experiments and assumed to be adequate for population needs.

If one takes iron as in table 3, RDinv corresponds to the population recommended intake PRI value of iron which is equal to 7mg/day according to the European Food Safety Authority. Consequently Rini,min is (7 x 7) = 49mg for the given duration Dg. The maximum chronic daily intake MCD for iron is not available from European Food Safety Agency. So Rini for iron will contain all values above 49mg, 49mg included, which can be written [49mg ; +∞[.

If one takes copper as in table 3, RDinv corresponds to the adequate intake AI value of copper which is 1,3mg/day according to the European Food Safety Authority.. Consequently Rini,min for copper for the given duration is (1,3 x 7) = 9,1mg. The maximum chronic daily intake MCD for copper given by the European Food Safety Agency is 5mg/day, so the Rini,max is (5 x 7) = 45mg for the given duration Dg. So Rini for copper over a period of 7 days contains all numbers lying between 9,1mg and 45mg.

The calculation of the recommended intake interval Rini of each nutrient is then identical as in the first embodiment. The recommended intake interval Rini values have been placed in table 3 thereafter.

Table 3:

| Nutrients | Rinv | MCD | Rini | | | Quantity derived from foodstuff quantity before step 1200/ day | NAinv | Quantity denved from foodstuff quantity after step 1200 / day | AAinv |
|---|---|---|---|---|---|---|---|---|---|
| | | | Rini, min | - | Rini, max | | | | |
| Carbohydrates (g) | 196,4 | 262 | 1374,8 | - | 1834 | 152,9 | 1070,3 | 180,0 | 1260 |
| Protein (g) | 66,0 | Not available | 462 | - | | 89,7 | 627,9 | 86,7 | 599.9 |
| Fat (g) | 38,0 | 67,8 | 266 | - | 474,6 | 83,6 | 585,2 | 76,5 | 535,5 |
| Fiber (g) | 25,0 | Not available | 175 | - | | 19,2 | 134,4 | 23,5 | 164,5 |
| Omega 3 a-linolenic acid(g) | 1,0 | Not available | 7 | - | | 2,5 | 17,5 | 2,9 | 20,3 |
| Omega 6 Linoleic acid (g) | 7,8 | 12 | 54,6 | - | 84 | 8,8 | 61,6 | 7.9 | 55,3 |
| Vit,A Retinol (μg) | 490,0 | 3000 | 3430 | - | 21000 | 243,9 | 1707,3 | 195,7 | 1369,9 |
| Vit,B1 Thiamin (mg) | 0,5 | Not available | 3,5 | - | | 1,9 | 13,3 | 1.9 | 13.3 |
| Vit,B2 Riboflavin (mg) | 1,3 | Not available | 9,1 | - | | 1,5 | 10,5 | 1,3 | 9,1 |

(continued)

| Nutrients | Rinv | MCD | Rini | | | Quantity derived from foodstuff quantity before step 1200/ day | NAinv | Quantity denved from foodstuff quantity after step 1200 / day | AAinv |
|---|---|---|---|---|---|---|---|---|---|
| | | | Rini, min | - | Rini, max | | | | |
| Vit,B3 Niacin (mg) | 9.5 | 35 | 66,5 | - | 245 | 21,1 | 147,7 | 18,4 | 128,8 |
| Vit,B5 Pantothenic acid (mg) | 5,0 | Not available | 35 | - | | 5,9 | 41,3 | 5,1 | 35,7 |
| Vit,B6 Pyridoxine (mg) | 1,3 | 25 | 9.1 | - | 175 | 2 | 14 | 2,1 | 14,7 |
| Vit,B7 Biotin (μg) | 40,0 | Not available | 280 | - | | 38,2 | 267,4 | 46.3 | 324,1 |
| Vit,B9 Folate (μg) | 250,0 | 1000 | 1750 | - | 7000 | 295,1 | 2065,7 | 487,7 | 3413,9 |
| Vit,B12 Cobalamin (μg) | 4,0 | Not available | 28 | - | | 5,8 | 40,6 | 4,4 | 30,8 |
| Vit,C Ascorbic acid (mg) | 80,0 | 2000 | 560 | - | 14000 | 108,5 | 759,5 | 165,9 | 1161,3 |
| Vitamin D (μg) | 15,0 | 100 | 105 | - | 700 | 4,3 | 30,1 | 8.9 | 62,3 |
| Vitamin E (mg) | 11,0 | 300 | 77 | - | 2100 | 10,4 | 72,8 | 13,7 | 95,9 |
| Vitamin K (μg) | 70.0 | Not available | 490 | - | | 75 | 525 | 139,7 | 977,9 |
| Copper (mg) | 1,3 | 5 | 9,1 | - | 35 | 1.4 | 9.8 | 1,9 | 13,3 |
| Iron (mg) | 7.0 | 45 | 49 | - | 315 | 12,4 | 86,8 | 15,8 | 110,6 |
| Iodine (μg) | 150 0 | 600 | 1050 | - | 4200 | 98,4 | 688,8 | 65,2 | 456,4 |
| Manganese (mg) | 3,0 | 11 | 21 - | - | 77 | 4,9 | 34,3 | 6,2 | 43,4 |
| Selenium (μg) | 70,0 | 300 | 490 | - | 2100 | 769 | 538,3 | 94,1 | 658,7 |
| Zinc (mg) | 8,0 | 40 | 56 - | - | 280 | 11,8 | 82,6 | 9,4 | 65,8 |
| Calcium (mg) | 750,0 | 2500 | 5250 | - | 17500 | 682,9 | 4780,3 | 664,9 | 4654,3 |
| Chloride (g) | 2,3 | 3,1 | 16,1 | - | 21 7 | 2,7 | 18,9 | 2,6 | 18,2 |
| Phosphorus (mg) | 550,0 | 4000 | 3850 | - | 28000 | 14094 | 9865,8 | 1471,4 | 10299,8 |
| Magnesium (mg) | 300,0 | Not available | 2100 | - | | 360,8 | 2525,6 | 472,9 | 3310.3 |
| Potassium (mg) | 3500,0 | Not available | 24500 | - | | 3323,6 | 23265,2 | 4163,4 | 29143,8 |
| Sodium (g) | 1,5 | 2 | 10,5 | - | 14 | 1,7 | 11,9 | 1,8 | 12,6 |

The present method can be a computer-implemented method. Thus, a data processing system comprising means for carrying out the process that has just been described in detail.

It can also be coded as a computer program or software comprising instructions which, when the program is executed by a computer, cause the computer to carry out the method according to the present invention. A data carrier signal carrying the computer program which, when the program is executed by a computer, cause the computer to carry out the method according to the present invention. The computer program can be a software application on the web and/or on mobile phones, thus facilitating users' access to a new and easy-to-use solution, to satisfy their nutritional needs through an appropriate personalized diet.

It can also be implemented in a computer-readable medium comprising instructions which, when executed by a computer, cause the computer to carry out the method described above.

The invention is obviously not limited to the examples which have just been described and numerous modifications can be made to these examples without departing from the scope of the invention. The invention, as it has just been described, achieves the goal it had set for itself, and makes it possible to propose for a subject to have at its disposal a list of foodstuffs, nutritional supplements and quantities of each foodstuff and nutritional supplements to be consumed, within a certain period of time obtained through a new process to meet its nutritional needs. The process is easier to implement than a daily menu that requires more time resources for purchasing, preparation, etc. which does not fulfill personalized needs based on a characteristic of the subject such as the nutrigenetic profile and does not consider personal food preferences of the subject.

The invention can therefore be useful for nutritionists, dietitians, fitness trainers, doctors specialized in diabetes, nutrition and metabolic diseases, and other specialists who will benefit from precise quantitative information about the necessary nutrients needed in nutritional management, they can offer nutritional solutions to their clients. The invention can also be useful for people who are interested in healthy eating, sportsmen, pregnant or breastfeeding women, overweight or obese people or individuals who want to prevent possible illnesses, will be able to feed according to the personalized nutritional profile.

**Claims**

1. Process of selecting foodstuffs (1000) for a diet of a subject that meets nutrients needs of the subject for a given duration (Dg), said process comprising

   - a step of calculating (1100) a recommended intake interval (Rini) for each nutrient of a plurality of nutrients for the given duration (Dg) according to at least one characteristic (CHA) of the subject,
   - a step of determining (1200) an actual intake value (NAinv) for each nutrient of the plurality of nutrients derived from subject's foodstuffs eating habits over an usual duration (Tu) which is equal to the given duration (Dg),
   - a step of comparing (1300) the actual intake value (NAinv) with respect to the recommended intake interval (Rini) for each nutrient of the plurality of nutrients,
   - if the actual intake value (NAinv) of at least one nutrient of the plurality of nutrients is outside its corresponding recommended intake interval (Rini), then proceed to a step of modifying (1400) quantity of foodstuffs for the diet in order to adjust the actual intake value (NAinv) of each nutrient of the plurality of nutrients within or below the corresponding recommended intake interval (Rini),
   - if at least one nutrient of the plurality of nutrients has an adjusted actual intake value (AAinv) below its corresponding recommended intake interval (Rini) after the step of modifying (1400), then proceed to a step of computing (1500) a quantity of nutrient supplement (Qns) of said one nutrient to be added to the adjusted actual intake value (AAinv) so that the quantity of nutrient supplement (Qns) of said one nutrient added to the adjusted actual intake value (AAinv) of said one nutrient falls into the corresponding recommended intake interval (Rini).

2. Process of selecting foodstuffs (1000) according to claim 1, during which the at least one characteristic (CHA) of the subject is a nutrigenetic profile of the subject and/or a physical trait chosen in the group comprising a mass, an age, a gender, a pregnancy state and/or a lactation state of the subject.

3. Process of selecting foodstuffs (1000) according to any of the preceding claims, during which the step of calculating (1100) the recommended intake interval (Rini) of each nutrient of the plurality of nutrients for the given duration (Dg) comprises a first substep of obtaining (1130) a recommended daily intake value (RDinv) for each nutrient and a second substep of creating (1160) the recommended intake interval (Rini) of each nutrient of the plurality of nutrients for the given duration (Dg) from the recommended daily intake value (RDinv) of each nutrient.

4. Process of selecting foodstuffs (1000) according to the preceding claim, during which the recommended intake interval (Rini) for each nutrient has a lower bound (Rini,min) which is equal to the recommended daily intake value (RDini) of the nutrient times the given duration (Dg).

5. Process of selecting foodstuffs (1000) according to the preceding claims 3 to 4, during which the recommended intake interval (Rini) for each nutrient has a upper bound (Rini,max) which is a maximum chronic daily intake (MCD) for the nutrient times the given duration (Dg).

6. Process of selecting foodstuffs (1000) according to any of the preceding claims, during which the step of determining (1200) the actual intake value (NAinv) for each nutrient of the plurality of nutrients derived from subject's foodstuffs eating habits comprises a first substep of evaluating (1230) quantities of actual foodstuffs eaten by the subject over the usual duration (Tu), and a second substep of converting (1260) the quantities of actual foodstuffs (Qaf) eaten by the subject into actual intake value (NAinv) for each nutrient of the plurality of nutrients.

7. Process of selecting foodstuffs (1000) according to any of the preceding claims, comprising a step of assessing (1600) foodstuff preferences of the subject.

8. Process of selecting foodstuffs (1000) according to the preceding claim, during which the step of assessing (1600) foodstuff preferences of the subject is conducted at the same time as the step of determining (1200) the actual intake value (NAinv) for each nutrient.

9. Process of selecting foodstuffs (1000) according to any of the preceding claims, comprising a step of representing (1900), in a single graph, the recommended intake interval (Rini) of each nutrient, and the adjusted actual intake value (AAinv) for each nutrient.

10. Process of selecting foodstuffs (1000) according to any of the preceding claims, comprising a step of transforming (1710) the computed quantity of nutrient supplement (Qns) into commercialized quantities used by the manufacturers of nutrient supplement product to identify the exact quantity to be purchased.

11. Process of selecting foodstuffs (1000) according to any of the preceding claims, comprising a step of purchasing (1720) modified quantity of foodstuffs for the diet and/or calculated quantity of nutrient supplement (Qns) for the given duration (Dg).

12. Process of selecting foodstuffs (1000) according to any of the preceding claims, comprising a step of proposing (1730) menus and/or recipes from the modified quantity of foodstuffs for the diet and calculated quantity of nutrient supplement within the given duration (Dg).

13. Process of selecting foodstuffs (1000) according to any of the preceding claims, comprising a step of monitoring (1800) the evolution of health of the subject during the given duration (Dg).

14. Process of selecting foodstuffs (1000) according to the preceding claim, during which the step of monitoring (1800) the evolution of the subject's health is made by basic biochemical analysis such as blood sample analysis and/or urine sample analysis.

15. Computer program comprising instructions which, when the program is executed by a computer, cause the computer to carry out the process of selecting foodstuffs (1000) according to any of the preceding claims.

[fig 1]

[fig 2]

Europäisches
Patentamt

European
Patent Office

Office européen
des brevets

# EUROPEAN SEARCH REPORT

Application Number

EP 20 20 0890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/371452 A1 (MAINARDI FABIO [CH]) 5 December 2019 (2019-12-05) * paragraphs [0081] - [0214] * ----- | 1-15 | INV. G16H20/60 |
| X | SHIHONO KARIKOME ET AL: "A system for supporting dietary habits", THE ICUIMC'10, ICUIMC '10 PROCEEDINGS OF THE 4TH INTERNATIONAL CONFERENCE ON UNIQUITOUS INFORMATION MANAGEMENT AND COMMUNICATION JANUARY 14-15, 2010, SKKU, SUWON, KOREA, ACM, KOREA, 14 January 2010 (2010-01-14), pages 1-6, XP058007363, DOI: 10.1145/2108616.2108684 ISBN: 978-1-60558-893-3 * the whole document * ----- | 1-15 | |
| X | CN 109 932 040 A (SUZHOU HENGDING TECH CONSULTING CO LTD) 25 June 2019 (2019-06-25) * abstract * ----- | 1-15 | |

TECHNICAL FIELDS SEARCHED (IPC)

G16H

The present search report has been drawn up for all claims

| Place of search | Date of completion of the search | Examiner |
|---|---|---|
| Munich | 26 March 2021 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 0890

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2021

| Patent document cited in search report | | Publication date | Patent family member(s) | | Publication date |
|---|---|---|---|---|---|
| US 2019371452 | A1 | 05-12-2019 | CN 109964280 A | | 02-07-2019 |
| | | | EP 3549139 A1 | | 09-10-2019 |
| | | | JP 2020504362 A | | 06-02-2020 |
| | | | US 2019371452 A1 | | 05-12-2019 |
| | | | WO 2018099838 A1 | | 07-06-2018 |
| CN 109932040 | A | 25-06-2019 | NONE | | |

EPO FORM P0459

For more details about this annex : see Official Journal of the European Patent Office, No. 12/82